# EUROPEAN PATENT APPLICATION

(11) **EP 2 075 343 A1**
(43) Date of publication of application: **01.07.2009**
(21) Application number: 07450240.2
(22) Date of filing: 21.12.2007
(51) Int. Cl.: C12Q 1/68

(54) **A method of diagnosing a progressive disease**

(71) Applicant: Mayer, Gert, 6020 Innsbruck (AT)
(72) Inventor: Mayer, Gert, 6020 Innsbruck (AT); Rudnicki, Michael, 6170 Zirl (AT); Eder, Susanne, 6091 Birgitz (AT); Schratzberger, Gabriele, 6020 Innsbruck (AT); Enrich, Julia, 6170 Zirl (AT); Perco, Paul, 1080 Vienna (AT)
(74) Representative: Redl, Gerda

(57) **Abstract**

The present invention relates to the method of prognosing or diagnosing a progressive disease comprising determining the amount of a marker selected from IL1RN, ISG15, LIFR, C6, IL32 or any combination thereof in a sample.

## Description

The present invention relates to a method for predicting the progression of chronic kidney disease by measuring a panel of biomarkers.

The increasing prevalence of patients on renal replacement therapy has become a major challenge for healthcare systems. Frequently, end stage renal disease (ESRD) is the terminal phase of a chronic process. A better understanding of the patho-physiology of progressive kidney disease could lead to the development of new treatment options which might be able to stabilize renal function and reduce the incidence of ESRD. In order to use new but also the already available drugs even more efficiently, it is also highly desirable to identify patients with an adverse renal prognosis in the early phases of the disease as not all subjects show a relentlessly progressive decline in renal function. In this context the magnitude of proteinuria has been suggested to be a useful risk marker, even though, on an individual basis, the discriminatory power is questionable. Other biomarkers such as apolipoprotein A-IV (APOA4), adiponectin (ADIPOQ), or fibroblast growth factor 23 (FGF23) have recently been proposed as alternative parameters to predict the course of disease.

It is a goal of the present invention to provide markers to identify patients with a chronic progressive disease.

Therefore, the present invention provides a method of diagnosing a progressive disease and/or assessing long term prognosis of a disease by determining the amount of a marker selected from IL1RN, ISG15, LIFR, C6, IL32 or any combination thereof in a sample. For the inventive method one of these markers can be detected, or a combination of any two, three, four, or five of these markers.

The inventive markers are: 1. C6 - Complement component 6 (UniGene: Hs.481992, GeneID: 729, GenBank: N59396): C6 is a component of complement cascade. It is part of the membrane attack complex which can insert into the cell membrane and cause cell to lyse. People with C6 deficiency are prone to bacterial infection.

2. IL32 - Interleukin 32 (UniGene: Hs.943, GeneID: 9235, GenBank: AA458965): This gene encodes a member of the cytokine family. The protein contains a tyrosine sulfation site, 3 potential N-myristoylation sites, multiple putative phosphorylation sites, and an RGD cell-attachment sequence. Expression of this protein is increased after the activation of T-cells by mitogens or the activation of NK cells by IL-2. This protein induces the production of TNFalpha from macrophage cells. Alternate transcriptional splice variants, encoding different isoforms, have been characterized.

3. ISG15 - ISG15 ubiquitin-like modifier (UniGene: Hs. 458485, GeneID: 9636, GenBank: AA406019 / AA120862).

4. IL1RN - interleukin 1 receptor antagonist (UniGene: Hs. 81134, GeneID: 3557, GenBank: T71181): The protein encoded by this gene is a member of the interleukin 1 cytokine family. This protein inhibits the activities of interleukin 1, alpha (IL1A) and interleukin 1, beta (IL1B), and modulates a variety of interleukin 1 related immune and inflammatory responses. This gene and five other closely related cytokine genes form a gene cluster spanning approximately 400 kb on chromosome 2. A polymorphism of this gene is reported to be associated with increased risk of osteoporotic fractures and gastric cancer. Four alternatively spliced transcript variants encoding distinct isoforms have been reported.

5. LIFR - leukemia inhibitory factor receptor alpha (UniGene: Hs.133421, GeneID: 3977, GenBank: AI820550): The leukemia inhibitory factor is a polyfunctional cytokine that affects the differentiation, survival, and proliferation of a wide variety of cells in the adult and the embryo. LIF action appears to be mediated through a high-affinity receptor complex composed of a low-affinity LIF binding chain (LIF receptor) and a high-affinity converter subunit, gp130. Both LIFR and gp130 are members of a family of cytokine receptors that includes components of the receptors for the majority of hematopoietic cytokines and for cytokines that affect other systems, including the ciliary neurotrophic factor, growth hormone and prolactin.

The inventive method can also include the step of obtaining the sample from a patient potentially suffering from a progressive renal disease. The progresssive nature of the disease is indicated if the amount of a marker or the combination of markers is increased at least 1.2 times the reference value of subjects not suffering from the progressive disease, preferably being healthy subjects or subjects suffering from a chronic non-progressive disease. In special embodiments the amount of IL1RN is at least 1.2, preferably at least 1.5, at least 1.8, at least 2, at least 3, at least 4, at least 5, at least 6, or at least 8 times the reference value, in particular as determined by PCR with either PPIA or GAPDH as endogenous controls or as determined by microarray analysis. In special embodiments the amount of ISG15 is at least 1.2, preferably at least 1.5, at least 1.8, at least 2, at least 3, at least 4, at least 5 or at least 6 times the reference value, in particular as determined by PCR with either PPIA or GAPDH as endogenous controls or as determined by microarray analysis. In special embodiments the amount of LIFR is at least 1.2, preferably at least 1.5, at least 1.8, at least 2 or at least 3 times the reference value, in particular as determined by PCR with either PPIA or GAPDH as endogenous controls or as determined by microarray analysis. In special embodiments the amount of C6 is at least 1.2, preferably at least 1.5, at least 1.7, at least 1.8 or at least 2 times the reference value, in particular as determined by PCR with either PPIA or GAPDH as endogenous controls or as determined by microarray analysis. In special embodiments the amount of IL32 is at least 1.2, preferably at least 1.5, at least 1.7, at least 1.8 or at least 2 times the reference value, in particular as determined by PCR with either PPIA or GAPDH as endogenous controls or as determined by microarray analysis. If more than one marker is detected, the comparison is made to each single reference value for each marker in the non-progressive disease or healthy reference itself. The inventive prognosis method can distinguish if a chronic disease is stable (i.e. the symptoms do not increase over a period of about at least or up to four, six, eight, ten months, one, two or three years after the sample was obtained) or is a progressive disease, i.e. the condition of the subject will increasingly suffer (e.g. over the same time span).

The inventive markers can be detected in any sample of a subject comprising said markers e.g. either as mRNA or expressed protein. The comparison with the reference value should be of the same sample type. In particular, the sample can be tissue, e.g. of a biopsy, blood, serum, plasma or a urine sample.

In preferred embodiments, determining the amount of the marker or any combination thereof comprises determining the expression of the marker(s), preferably by determining the mRNA concentration of the marker(s). To this extend, mRNA of the sample can be isolated (if necessary after adequate sample preparation steps, e.g. tissue homogenisation) and hybridized with marker specific probes, in particular on a microarray platform with or without amplification, or primers for PCR-based detection methods, e.g. PCR extension labelling with probes specific for a portion of the marker mRNA. In preferred embodiments the marker(s) or a combination thereof is (are) determined by microarray hybridization with IL1RN, ISG15, LIFR, C6, IL32 specific probes or by PCR.

In further embodiments the amount of a marker or any combination thereof is determined by the protein concentration of the marker(s), e.g. with marker specific antibodies or binding partners. The binding event can, e.g., be detected by competitive or non-competitive methods, including the use of labelled marker specific moieties (e.g. antibodies) or labelled competitive moieties (including a labeled marker standard) which compete with marker proteins for the binding event.

The condition which can be detected with the inventive methods is in particular a progressive renal disease, which can e.g. be determined by using a kidney biopsy sample and also by detecting the markers in serum, blood, plasma and urine by comparing reference values of non-progressive renal disease values or from healthy subjects.

The subject can, e.g., be any mammal, in particular a human, but also selected from mouse, rat, hamster, cat, dog, horse, cow, pig, etc.

In further embodiments the renal disease is selected from various nephropathies, including but not being limited to IgA nephropathy, non IgA mesangioproliferative glomerulonephritis, membranoproliferative glomerulonephritis, any postinfectious glomerulonephritis, focal-segmental glomerulosclerosis, minimal change disease, membranous nephropathy, lupus nephritis of any kind, vasculitides with renal involvement of any kind, any other systemic disease leading to renal disease including but not being limited to diabetes mellitus, hypertension or amyloidosis, any hereditary renal disease, any interstitial nephritis and renal transplant failure.

In a further aspect the present invention provides a set of at least two different marker specific moieties, e.g. more than two, three, four, or five marker specific moieties, wherein the markers are selected from IL1RN, ISG15, LIFR, C6 or IL32. Marker specific moieties are substances which can bind to or detect at least one of the markers for a detection method described above and are in particular marker protein specific antibodies or antibody fragments, such as Fab, F(ab), F(ab)', Fv, scFv, or single chain antibodies. The marker specific moieties can also be selected from marker nucleotide sequence specific oligonucleotides, which specifically bind to a portion of the marker sequences (e.g. mRNA or cDNA) or are complementary to such a portion in the sense or complementary anti-sense (cDNA complement-ary strand) orientation. For easy detection the moieties are preferably labelled, such as by optical, including fluorescence, and radioactive labels.

The present invention is further illustrated by the following figures and examples without being limited thereto.

### Figures:

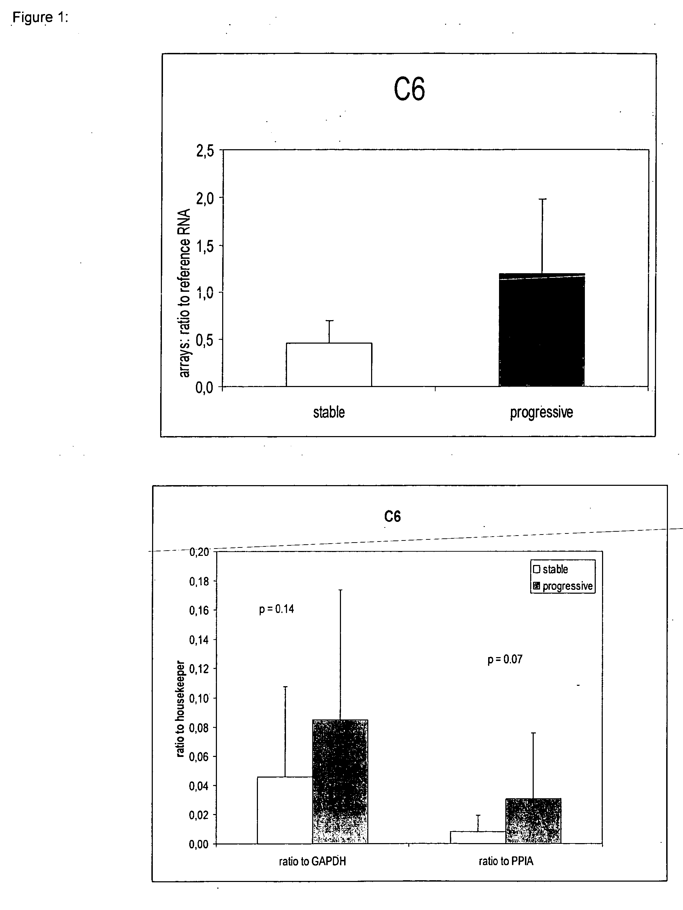

Fig. 1: Results for C6: Arrays: 14 SD vs 7 PD (fold-change for N59396: 2.27), rtPCR: 11 SD vs 9 PD (fold-change to PPIA: 3.88, fold-change to GAPDH: 1.85)

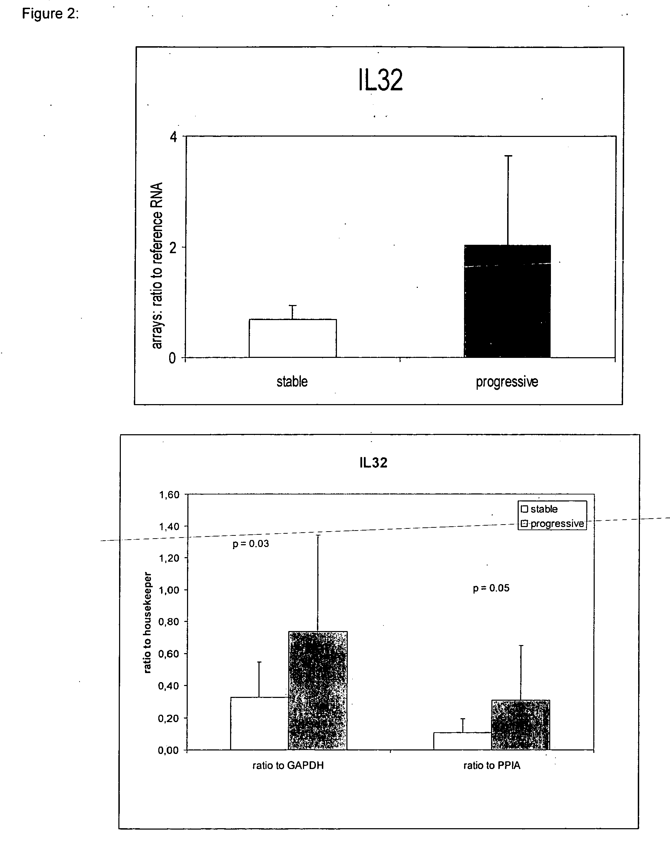

Fig. 2: Results for IL32: Arrays: 14 SD vs 7 PD (fold-change for AA458965: 2.78), rtPCR: 11 SD vs 9 PD (fold-change to PPIA 2.86 fold-change to GAPDH 2.25)

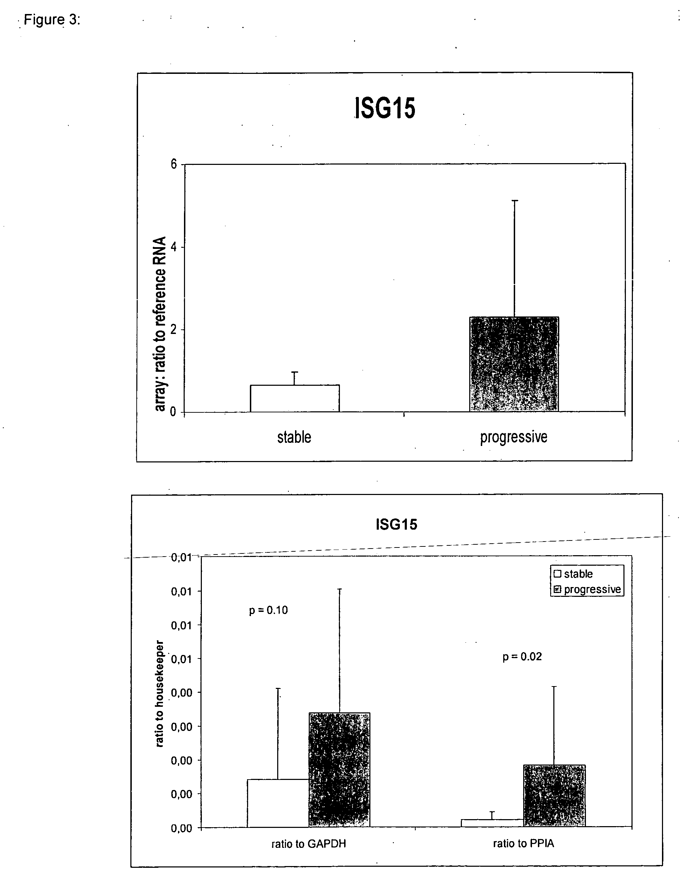

Fig. 3: Results for ISG15: Arrays: 14 SD vs 7 PD (fold-change for AA406019/AA120862: 2.79 and 2.19), rtPCR: 11 SD vs 10 PD (fold-change to PPIA: 8.27, fold-change to GAPDH: 2.40)

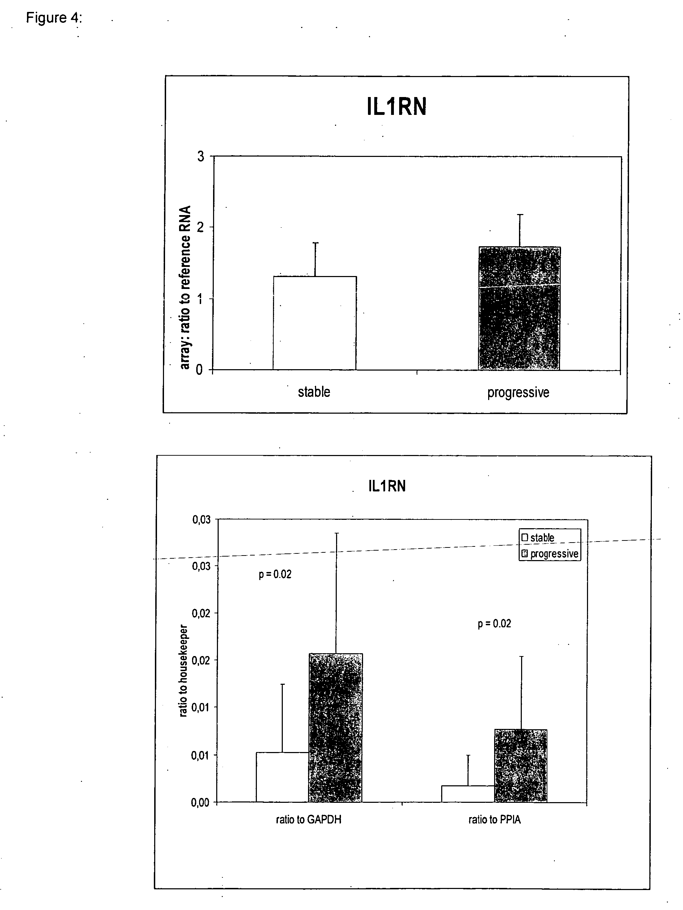

Fig. 4: Results for IL1RN: Arrays: 14 SD vs 7 PD (fold-change for T71181: 1.69), rtPCR: 11 SD vs 10 PD (fold-change to PPIA: 4.40, fold-change to GAPDH: 2.98)

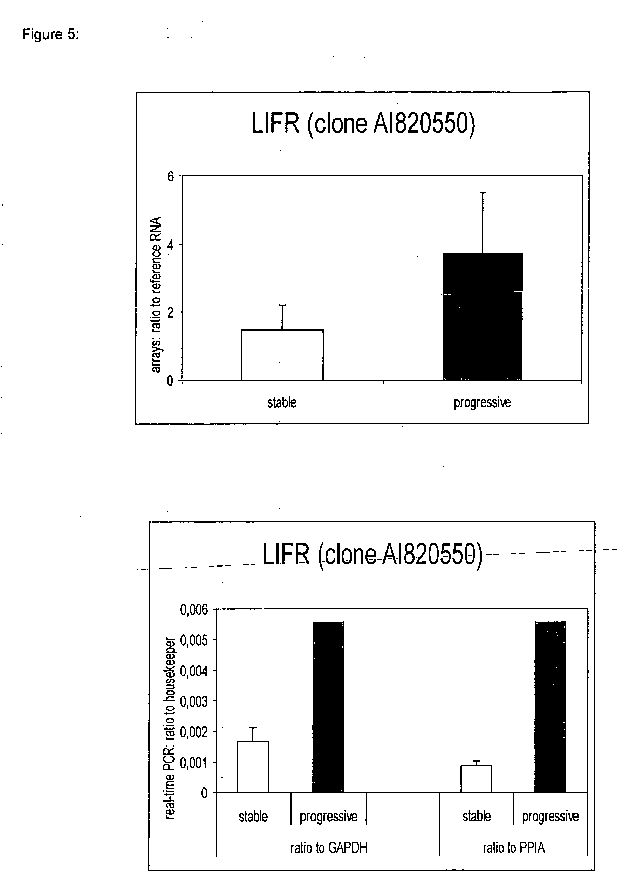

Fig. 5: Results for LIFR: Arrays: 14 SD vs 7 PD (fold-change for AI820550: 2.46), rtPCR: 3 SD vs 3 PD (fold-change to PPIA: 2.64, fold-change to GAPDH: 3.47)

### Examples:

### Example 1: Patient Samples

34 kidney biopsies obtained from patients with proteinuric renal diseases during their routine diagnostic workup were used. The histological diagnoses were IgA nephropathy (IGAN) in 14, focal-segmental glomerulosclerosis (FSGS) in 5, minimal change disease (MCD) in 3, lupus nephritis WHO class IV (SLE) in 4 and anti-GBM rapidly progressive glomerulonephritis (RPGN) in 2 patients. One subject each suffered from membranous nephritis (MN), membranoproliferative nephritis (MPGN), p-ANCA positive microscopic polyangiitis (MPA), vascular nephropathy (VNP), MPGN with purpura Schoenlein-Henoch and diabetic nephropathy (DN). Microarray-based gene expression profiling was performed in 21 of these patients' samples, while real-time PCR validation experiments were performed in 20 with 7 samples being analysed by both microarray and real-time PCR.

Based on the course of excretory kidney function during a 2 to 46 month follow-up period after biopsy, this cohort was split into subjects with "stable disease (SD)" and "progressive disease (PD)". Patients were defined as progressive when they experienced a persistent rise of at least 50% in serum creatinine during follow-up, or when they reached end-stage renal disease (n=14). All other patients were defined as stable (n=20). Light microscopy of the biopsies was performed using hematoxylin/eosin and periodic-acid-Schiff (PAS) or Pearse-stained sections. Scoring of tubulointerstitial fibrosis was performed by an independent pathologist following a semiquantitative grading system: 0 no fibrosis; 1: < 10%; 2: 10 - 25; 3: 25 - 50; 4: 50 - 75; 5: 75%. Material used for microarray studies was obtained from cryo-cut sections.

### Example 2: RNA isolation and microarray hybridization

Processing of frozen sections, isolation of proximal tubular cells, isolation of total RNA, quality control by RT-PCR and T-7 based linear amplification of RNA were performed as recently described by Rudnicki et al. (Kidney Int 2007; 71: 325-35). The linearity and reproducibility of two rounds of RNA amplification for microarray experiments using RiboAmp™ RNA amplification kit (Arcturus, Mountain View, CA, USA) has also been evaluated. Two rounds amplified Universal Human Reference RNA™ (Stratagene, La Jolla, CA, USA) was used as reference RNA in all experiments. Using the CyScribe cDNA Post Labelling Kit (formerly Amersham Biosciences, now GE healthcare life sciences, Piscataway, NJ, USA), 1 µg of amplified sample RNA (Cy5, red) and 1µg of amplified reference RNA (Cy3 green) were labeled and cohybridized to the arrays as described by Rudnicki et al. (Nephon Exp Nephrol 2004; 97: e86-95). cDNA microarrays were obtained from the Stanford Functional Genomics Facility (www.microarray.org/sfgf/). The arrays contained 41792 spots, representing 30325 genes assigned to a UniGene cluster and 11467 ESTs. All samples were processed in technical duplicates. Arrays were scanned using a GenePix 4000B microarray scanner and the images were analyzed with the GenePix Pro 4.0 software (Axon Instruments, Union City, CA).

### Example 3: Statistical analysis and selection of putative biomarkers

Signals showing intensity signal over background values < 2.5 in either channel were excluded and the analyses were focused on genes with valid data in at least 80% of processed samples, leaving 19921 cDNA clones in the analysis dataset. Gene expression values of technical array replicates were averaged. A two-sample t-test (p < 0.05) and the two-fold-change criterion were used to identify differentially expressed genes (DEGs) when comparing both patient cohorts.

The subcellular location of DEGs was determined using data stored in the SwissProt database, and secreted proteins were identified. The secreted DEGs showing the highest fold change values were selected for validation via real-time PCR experiments.

### Example 4: Validation via real-time PCR

The TaqMan™ PreAmp Master Mix (Ambion, Austin, TX, USA) together with the respective TaqMan™ probes (vide infra) was used for 300-400fold amplification of the original RNA as the laser-capture microdissection of proximal tubular cells from frozen sections yields about 1 ng of total RNA per sample. In all real-time PCR experiments GAPDH (Hs99999905_m1) and PPIA (cyclophilin A; Hs99999904_m1) were used as endogenous controls. Based on the ratios to housekeeper genes (i.e. 2 exp delta ct) a fold-change between stable and progressive disease patients was calculated and compared to the fold-change values obtained by microarray analysis.

### Example 5: Microarray analysis

Of the 113 DEGs upregulated in the progressive disease samples six had protein isoforms which were secreted according to information stored in the SwissProt database. These genes are the complement component 6 (C6), interleukin 32 (IL32), ISG15 ubiquitin-like modifier (ISG15), the leukemia inhibitory factor receptor alpha (LIFR), transferring (TF), and TIMP metallopeptidase inhibitor 1 (TIMP1). In addition, the interleukin 1 receptor antagonist was included due to its very low p-value of < 0.0001.

| GeneName | GeneSymbol | p-value | Fold-change (array) |
|---|---|---|---|
| TIMP metallopeptidase inhibitor 1 | TIMP1 | 0.00010 | 4.24 |
| Transferrin | TF | 0.00815 | 3.79 |
| ISG15 ubiquitin-like modifier | ISG15 | 0.00575 | 2.79 |
| Interleukin 32 | IL32 | 0.01162 | 2.78 |
| Complement component 6 | C6 | 0.00036 | 2.27 |
| Leukemia inhibitory factor receptor alpha | LIFR | 0.01169 | 2.02 |
| Interleukin 1 receptor antagonist | IL1RN | 0.00005 | 1.69 |

### Example 6: Validation via real-time PCR

The upregulation of five out of the seven selected biomarkers could be validated in rtPCR experiments. Two markers showed a downregulation, namely TF and TIMP1, i.e. these proteins were not confirmed in rtPCR.

| GeneName | Gene- Symbol | p-value | Fold-change (array) | Fold-change (rtPCR to PPIA) | Fold-change (rtPCR to GAP-DH) |
|---|---|---|---|---|---|
| Interleukin 1 receptor antagonist | IL1RN | 0.00005 | 1.69 | 4.40 | 2.98 |
| ISG15 ubiquitin-like modifier | ISG15 | 0.00575 | 2.79 | 8.27 | 2.40 |
| Leukemia inhibitory factor receptor alpha | LIFR | 0.01169 | 2.02 | 2.64 | 3.47 |
| Complement component 6 | C6 | 0.00036 | 2.27 | 3.88 | 1.85 |
| Interleukin 32 | IL32 | 0.01162 | 2.78 | 2.86 | 2.25 |
| TIMP metallopeptidase inhibitor 1 | TIMP1 | 0.00010 | 4.24 | 1.11 | -1.35 |
| Transferrin | TF | 0.00815 | 3.79 | 1.28 | -2.76 |

## Claims

1. A method of prognosing or diagnosing a progressive disease comprising determining the amount of a marker selected from IL1RN, ISG15, LIFR, C6, IL32 or any combination thereof in a sample.

2. The method of claim 1, comprising the step of obtaining the sample from a patient potentially suffering from a progressive renal disease, and wherein the disease is indicated if the amount of a marker or the combination of markers is increased at least 1.2 times the reference value of subjects not suffering from the progressive disease, preferably being healthy subjects or subjects suffering from a chronic non-progressive disease.

3. The method of claim 1 or 2, **characterized in that** the sample is a tissue, blood, serum, plasma or urine sample.

4. The method of any one of claims 1 to 3, **characterized in that** determining the amount of a marker selected from IL1RN, IS-G15, LIFR, C6, IL32 or any combination thereof comprises determining the expression of the marker(s), preferably by determining the mRNA concentration of the marker (s).

5. The method according to any one of claims 1 to 3, **characterized in that** determining the amount of a marker selected from IL1RN, ISG15, LIFR, C6, IL32 or any combination thereof comprises determining the protein concentration of the marker(s).

6. The method according to any one of claims 1 to 5, **characterized in that** the disease is a progressive renal disease.

7. The method according to any one of claims 1 to 6, **characterized in that** the sample is a kidney biopsy sample.

8. The method according to claim 6 or 7, **characterized in that** the renal disease is selected from IgA nephropathy, non IgA mesangioproliferative glomerulonephritis, membranoproliferative glomerulonephritis, any postinfectious glomerulonephritis, focal-segmental glomerulosclerosis, minimal change disease, membranous nephropathy, lupus nephritis of any kind, vasculitides with renal involvement of any kind, any other systemic disease leading to renal disease including but not being limited to diabetes mellitus, hypertension or amyloidosis, any hereditary renal disease, any interstitial nephritis and renal transplant failure.

9. The method according to any one of claims 1 to 8, **characterized in that** the amount of a marker selected from IL1RN, ISG15, LIFR, C6, IL32 or a combination thereof is determined by microarray hybridization with IL1RN, ISG15, LIFR, C6, IL32 specific probes or by PCR.

10. A set of at least two marker specific moieties, wherein the markers are selected from IL1RN, ISG15, LIFR, C6 or IL32.

11. Set according to claim 10, **characterized in that** the moieties are IL1RN, ISG15, LIFR, C6 or IL32 protein specific antibodies or antibody fragments.

12. Set according to claim 10, **characterized in that** the moieties are IL1RN, ISG15, LIFR, C6 or IL32 nucleotide sequence specific oligonucleotides.

13. Set of any one of claims 10 to 12, **characterized in that** the moieties are labelled.
